# EUROPEAN PATENT APPLICATION

(11) **EP 4 325 386 A1**
(43) Date of publication of application: **21.02.2024**
(21) Application number: 22210895.3
(22) Date of filing: 01.12.2022
(51) Int. Cl.: G06F 21/57, G06T 1/00, G06T 7/00, G06T 7/80, G16H 30/00, H04L 9/32, H04L 9/00, G06F 8/71, G06F 8/60

(54) **IMAGING DEVICE CONFIGURATION VERIFICATION**

(30) Priority: 19.08.2022 WO PCT/CN2022/113588
(71) Applicant: Koninklijke Philips N.V., 5656 AG Eindhoven (NL)
(72) Inventor: LARMUSEAU, Adriaan Joris H., 5656AG Eindhoven (NL)
(74) Representative: Philips Intellectual Property & Standards

(57) **Abstract**

An imaging system provider provides updates to an imaging facility. These updates are referred to as configurations, and each configuration has an associated date-time. The provider stores each configuration and its date-time in a blockchain. When the imaging facility acquires an image of a patient, the image is watermarked with an identification of the configuration of the imaging system at the time the image was acquired. When the patient wants to verify whether the image was taken using the most up to date configuration, the patient submits an identification of the image to a verification system. The verifier submits a request for proof from the imagining facility. The imaging facility compares the watermarked information and the information in the block chain, and provides the proof to the verification system, which verifies the proof. To assure privacy and security, a zk-SNARK is used for the proving and verification processes.

## Description

### FIELD OF THE INVENTION

This invention relates to the field of medical imaging technology, and in particular to an automated system that verifies that a medical facility's imaging devices are operated using the most currently available security configurations.

### BACKGROUND OF THE INVENTION

"In 2018, clinics and hospitals were hit with numerous attacks leading to significant data breaches and interruptions in medical services. An attacker with access to medical records can do much more than hold the data for ransom or sell it on the black market... An attacker may perform this act in order to stop a political candidate, sabotage research, commit insurance fraud, perform an act of terrorism, or even commit murder."¹

To combat these malicious attacks, improvements to security protocols are continually being developed. Of particular note, new releases of imaging software and associated components occur regularly as new security threats are identified. However, the protection against these new threats can only be realized if the clinic or hospital continually update their imaging systems with these new security releases.

"In 2016, Stites and Pianykh performed a scan through the World Wide Web of networked computers and devices and showed that there were 2,774 unprotected radiology or DICOM servers worldwide, most of them located in the United States."²

### SUMMARY OF THE INVENTION

It would be advantageous to provide a system and method that enables an efficient and effective verification that an imaging device had the latest security configuration installed when a particular image or set of images was produced. As used herein, 'security configuration' may include an identification of the version of the imaging software, as well as an identification of a configuration of components associated with the imaging system that the software provider provides for improved security.

In embodiments of this invention, as a software provider releases a new security configuration for an imaging system, a timestamp of the configuration's release is stored in a blockchain. At the imaging facility, as each image is produced, it is watermarked with a watermark that includes an image timestamp and the security configuration of the imaging device at the time that the image was produced.

A verification system receives a verification request from a user, requesting verification that, at the time a particular image was produced by a imaging system, the imaging system was operating with an up-to-date security configuration. The user may submit a hash of the user's image to the verification system to identify the subject image. The verification system submits this verification request to the imaging facility, and the imaging facility provides 'proof' that the imaging system was properly configured by comparing the image timestamp to the record of configuration releases recorded in the blockchain. Upon receipt, the verification system assesses the proof in view of the record in the blockchain to provide verification to the user that the imaging system was, or was not, operating with an up-to-date security configuration.

In a preferred embodiment, a zero-knowledge Succinct Non-Interactive Arguments of Knowledge (zk-SNARK) system is used by the imaging facility to provide the proof, and by the verification system to verify the proof. A third party, typically the imaging system provider, generates the prover and a prover-key that is used by the imaging facility, and the verifier and a verifier-key that are used by the verification system.

The use of this invention will improve the security of medical imaging technology by enabling a rapid assessment of an imaging facility's security measures for maintaining up-to-date security configurations of their imaging systems. In addition to verifying image security configuration requests from individual users, the image security verification system of this invention may be used, for example, by government and insurance agencies using random images over time to assess the imaging facility's compliance with security regulations.

As noted above, the use of this invention will also enhance the security of individual images by notifying a patient that the particular image may have been susceptible to attacks because of the lack of adequate security configuration updates at the medical facility. Upon such notification, the patient may choose to have new images taken using a more secure configuration of the imaging system.

In like manner, in embodiments that use zk-SNARK provers and verifiers, the security of the imaging facility is enhanced by eliminating disclosure of the particular security configurations in place at any given time. This may be of significant importance in 'sensitive' facilities, such as military field hospitals, because knowledge of a facility's security configuration can facilitate the development and/or deployment of attacks that are targeted to such security configurations.

These advantages, and others, may be achieved with minimal operational overhead by embodying the verification system as a "smart contract" that automates the entire image security configuration validation process in response to the validation request from the user or authorized agent.

### BRIEF DESCRIPTION OF THE DRAWINGS

The invention is explained in further detail, and by way of example, with reference to the accompanying drawings wherein:
FIG. 1 illustrates an example security configuration image verification system.
FIG. 2 illustrates an example flow diagram for verifying the security configuration of an image.
FIG. 3 illustrates an example flow diagram for determining whether the security configuration of an imaging system was up-to-date when the image was taken.
FIGs. 4A-4B illustrate an example zk-SNARK system.
FIGs. 5A-5B illustrate an example flow diagram of a security configuration image verification system using a zk-SNARK system.
FIG. 6 illustrates an example flow diagram of a smart contract embodiment of a security configuration image verification system using a zk-SNARK system.

Throughout the drawings, the same reference numerals indicate similar or corresponding features or functions. The drawings are included for illustrative purposes and are not intended to limit the scope of the invention.

### DETAILED DESCRIPTION OF THE EMBODIMENTS

In the following description, for purposes of explanation rather than limitation, specific details are set forth such as the particular architecture, interfaces, techniques, etc., in order to provide a thorough understanding of the concepts of the invention. However, it will be apparent to those skilled in the art that the present invention may be practiced in other embodiments, which depart from these specific details. In like manner, the text of this description is directed to the example embodiments as illustrated in the Figures, and is not intended to limit the claimed invention beyond the limits expressly included in the claims. For purposes of simplicity and clarity, detailed descriptions of well-known devices, circuits, and methods are omitted so as not to obscure the description of the present invention with unnecessary detail.

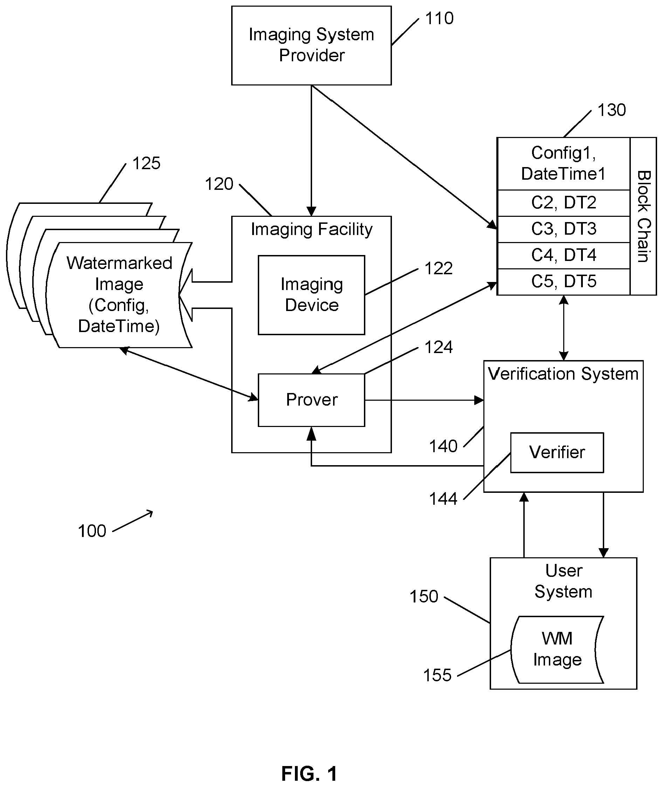

FIG. 1 illustrates an example security configuration image verification system 100 comprising an imaging system provider 110, an imaging facility 120, and a verification system 140.

The imaging system provider 110 provides updates associated with the imaging system to the various imaging facilities that use the provider's imaging system. One such imaging facility 120 is illustrated in FIG. 1, but there could be hundreds or thousands of such imaging facilities. These updates include, for example, software updates that are to be applied to the imaging device 122 at the facility 120, as well as updates to the configuration of the imaging device 122 and other components (not illustrated) at the facility to facilitate effective, efficient, and secure deployment of the provided imaging device.

As noted above, medical imaging facilities have been the target of malicious attacks, wherein attackers have gained unauthorized access to either the imaging equipment itself or the images produced by this equipment. This unauthorized access can lead to harm to the patients whose images are accessed, including, for example, modifications to the image to add lesions to the image that do not actually exist, or remove actual lesions from the image, or combinations of both. These modifications will mislead the medical professionals treating the patient, potentially harming or killing the patient when steps are taken to treat the non-existent added lesions or to ignore treatment of the erased lesions. Other consequences of unauthorized access to medical images are also possible.

In like manner, the operation of the medical imaging devices at the imaging facility may be tampered with, again with the possibility of bogus additions and deletions being made to the image as it is being created.

In response to the threat of unauthorized access to either the imaging devices or the stored images, the aforementioned updates provided by the imaging system providers may include updates that are in response to known or suspected threats to the security of the imaging systems. Vulnerabilities to software attacks may be addressed by providing updated software that increase protection from such vulnerabilities. Vulnerabilities to other attacks may be addressed by modifications to the components that interact with the imaging system, such as the operating system and/or networking system in which the imaging system operates.

For ease of reference, the term "security configuration" is used herein to identify any and all revisions to the imaging system and components that interact with the imaging system that the imaging system provider 110 provides to the imaging facility 120.

In accordance with aspects of this invention, each time the imaging system provider 110 creates and releases a new security configuration, the imaging system provider 110 enters the new security configuration and the time of release to a blockchain 130. The stored security configuration 130 may identify, for example, a set of "version numbers" associated with the hardware and/or software that represent the most recent configuration identified by the imaging system provider at the time of release of the particular security configuration. By storing these configuration settings and the time of each release on a blockchain, a secure record is assured, because any attempted change of the stored information will be immediately recognizable.

Correspondingly, each time the imaging facility 120 creates an image of a patient, the image 125 is 'watermarked' with an identification of the date-time that the image was acquired, as well as an identification of the security configuration of the imaging system at that time. Other identification information may also be included in the watermark, including, for example, an identifier of the patient, an identifier of the imaging facility, and so on. The watermarking process will typically be provided by the imaging system provider 110, and may be embodied in the imaging device 122. The imaging facility 120 may provide the patient with a copy of the watermarked image, either at the time the image was taken, or upon a subsequent request.

Some time after the image is taken, the patient and/or other authorized agent may have reason to question whether the imaging facility was operating with an up-to-date security configuration at the time that the image was created. When the patient presents the image to a medical professional at a different facility, the medical professional may request assurance that the particular medical image is trustworthy, wherein trustworthy is based on whether the particular imaging system was operating with the appropriate (i.e. up-to-date) security configuration.

In like manner, if the use of the particular image leads to harm to the patient due to a malicious act, the imaging facility may wish to provide proof that it was operating the imaging system with an up-to-date security configuration, in order to avoid liability.

Of particular note, with the ability to easily and/or automatically determine whether or not an imaging facility was operating with an up-to-date securing configuration when each image is acquired, operators of the imaging facilities will have a (self-interest) incentive to assure that when each new security configuration is provided by the imaging system provider it is immediately applied to their imaging systems. Also, the ability to easily and/or automatically determine whether or not an imaging facility was operating with an up-to-date securing configuration when each image is acquired may facilitate the determination of whether an imaging facility is consistently in conformance with security regulations or standards by requesting security configuration validation of random images over time.

As noted above, the system 100 includes a verification system 140. The verification system 140 receives a request for verification of a watermarked image 155 from a user system 150. The verification system 140 may be located at the imaging facility 120, but an independent verification system 140 will be able to provide image security verification for images from multiple imaging facilities. A verification system 140 that is independent of the imaging facility 120 may also instill confidence in the user that the resultant verification is trustworthy.

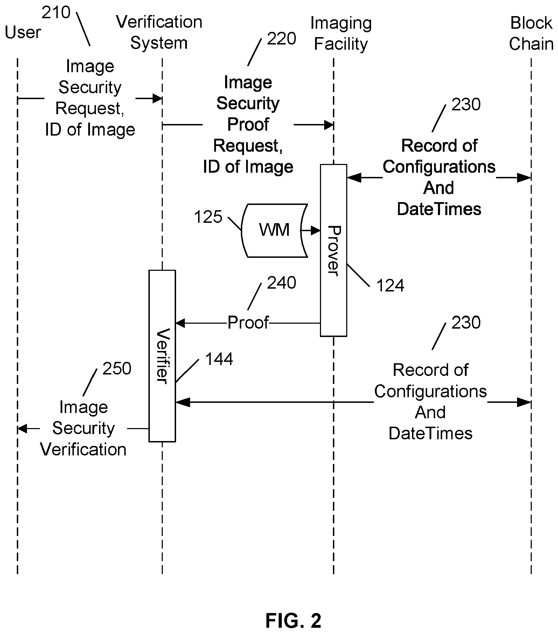

FIG. 2 illustrates an example flow diagram of the image security verification process in embodiments of this invention. The columns represent the actions by the User, the Verification system, the Imaging facility, and the Blockchain. Time is represented by the occurrence of each action in the vertical direction.

At 210, the user communicates a request 210 to the verification system. This request includes an identification of the image for which a security confirmation is requested.

At 220, the verification system requests a 'proof' from the imaging facility that the imaging system at the facility was using an up-to-date security configuration when the identified image was acquired.

A prover 124 at the imaging facility accesses the blockchain to access the provider's record of the date-time of each release of an updated security configuration 230, and decodes the watermark of the identified image 125 to determine the image date-time and the security configuration present at the imaging system when the image 125 was acquired. The prover compares the image security configuration at the image date-time to the record of the provider security configurations to determine whether the image security configuration was an up-to-date provider security configuration at the image date-time.

The prover 124 provides this proof 240 to the verifier 144. Depending upon the nature of this proof 240, the verifier 144 may also access the record of provider security configurations in the blockchain, as detailed further below.

Based on the verification of the proof from the imaging facility, the verification system notifies the user of the results of the image security verification 250.

Returning to the example embodiment of FIG. 1, in embodiments of this invention, the user (patient and/or authorized agent) 150 identifies the particular image 155 in question to the verification system. This identification can take a variety of forms, depending on tradeoffs regarding ease of use, authentication, unambiguity, communication resources, and so on. In some embodiments, for example, the user may need only provide an identification of the imaging facility, the date of the imaging, the patient's name, etc., and the verification facility 140 forwards this information to the particular imaging facility 120. In other embodiments, the user may need to provide a copy of the watermarked image 155 to the verification system 140 for forwarding to the medical facility.

In a preferred embodiment, the user 150 may provide a hash of the watermarked image 155 to the verification system 140 for forwarding to the imaging facility 120. Providing a hash of the image 155 has several advantages. Typically, a hash of an image is substantially smaller than the image itself, reducing communication resource requirements. Also, medical imaging facilities often use the hash of each image to index each image in their storage, and providing the hash of the user's image 155 will facilitate retrieval of the image at the imaging facility. A matching hash also assures that the user's image 155 has not been modified and, consequently is identical to the image 125 at the imaging facility 120.

Upon receipt of an image security verification request from the verification system 140, a prover 124 at the imaging facility provides 'proof' to the verification system 140 as to whether it was operating with an up-to-date security configuration.

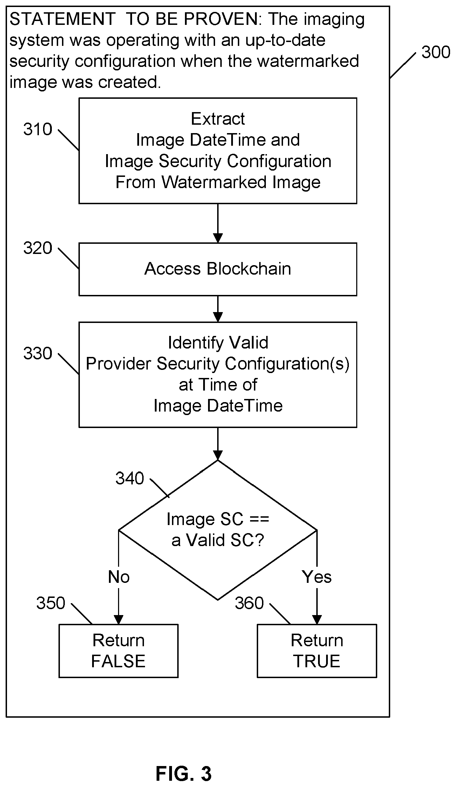

Flow diagram 300 in FIG. 3 provides an example of how a prover may determine whether or not the image system was using an up-to-date security configuration at the time the particular image was acquired.

At 310, the prover extracts the date-time that the image was taken, and the security configuration that was in place when the image was taken, by decoding the watermarked information in the image.

At 320, the prover accesses the blockchain, and at 330, the prover determines the latest security configuration(s) that were released to the imaging facility prior to the date-time of the image, as recorded by the image system provider in the blockchain.

There may be multiple up-to-date configurations associated with the date-time of the image, because, for example, it would likely be impossible for an imaging system to incorporate the latest release of the security configuration within minutes of the release from the provider. That is, each release will have a span of time for which it would be considered "up-to-date", wherein that span of time may overlap at least a portion of a subsequent release's date-time. For ease of reference, the term 'valid' security configuration is used herein to define a security configuration that is considered to be up-to-date at a given date-time.

The end time of this span of 'validity' for each security configuration may be included in the subsequent release's blockchain information (e.g. "Prior security configuration validity expires at xxx date-time"), or it may be a predefined relative end time (e.g. "Each security configuration is valid from its release date-time until n days after the release of the next security configuration"). Other schemes for defining the effective time span of each security configuration may also be used.

At 340, the prover compares the image security configuration from the watermark information to the valid provider security configuration(s) from the blockchain. If the image security configuration matches a valid provider security configuration for the date-time of the image, the prover determines that the imaging system was operating with an up-to-date security configuration (360 TRUE); otherwise, the prover determines that the imaging system was not operating with an up-to-date security configuration (350 FALSE).

Referring back to FIG. 1, the imaging facility 120 provides this determination to the verification system 140, and the verification system 140 notifies the user 150 of this determination. The verification system 140 preferably includes a verifier 144 that serves to assure the user 150 that the determination is valid. This verification may take a variety of forms, depending upon, for example, the degree of trust between the verification system 140 and the imaging facility 120. In some embodiments, for example, the imaging facility 120 may be part of a 'trusted network' of imaging facilities, wherein each imaging facility is operated in conformance with a set of standards and operating conditions, including, for example, regular inspections and monitoring practices. In such a case, the verifier 144 may only need to ascertain that the imaging facility's credentials in the trusted network are valid.

In other embodiments, the prover 124 may be required to disclose the image date-time and security configuration to the verifier 144, and the verifier 144 confirms that the image security configuration matches a valid provider security configuration at the date-time of the image. In some embodiments, the prover 124 may be required to prove to the verifier 144 that the identified image date-time and security configuration accurately reflect the watermarked information in the image.

An example embodiment that addresses these potential trust issues between the verification system 140 and the imaging system 120 is presented further below, based on a zk-SNARK prover and verifier.

The acronym zk-SNARK corresponds to zero-knowledge Succinct Non-interactive Arguments of Knowledge. In the zk-SNARK protocol, a prover determines whether a statement is true or false, and a verifier that confirms that the prover has presented sufficient proof of that determination. The zero-knowledge aspect of zk-SNARK process means that the proof provides no information regarding the basis of the determination.

In the context of this invention, the statement to be proved may be "the imaging system was operating with an up-to-date security configuration at the time that the subject image was taken". When the prover provides "proof' that the statement is true, this proof will reveal nothing about the particular security configuration that was in place at the time the image was taken. However, the "proof' will be sufficiently secure to enable the verifier to ascertain that the prover executed the appropriate process to make this determination, as detailed further below.

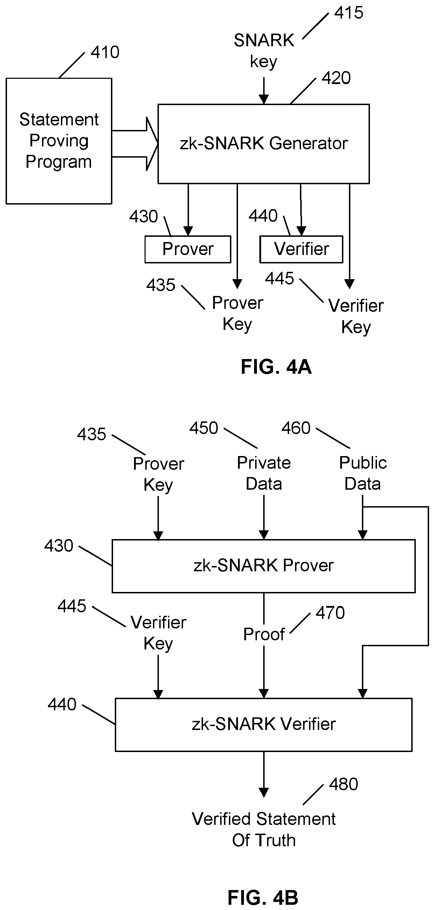

A zk-SNARK system includes three components: a generator, a prover, and a verifier, as illustrated in FIGs. 4A-4B. These components are typically provided by a trusted third party; in the context of this invention, these components would be provided by the imaging system provider.

The generator 420 receives a secret parameter (SNARK key 415) and a program 410 (such as the program 300 of FIG. 3), and creates the prover 430 and the verifier 440. The prover 430 corresponds to a secure encoding of the given program 410 in a manner that provides a proof to the verifier 440 that the program was executed properly. The generator 420 also creates a corresponding prover key 435 and corresponding verifier key 445 that are used by the prover and verifier, respectively. The SNARK key 415 ensures that the generated programs 430, 440 and keys 435, 445 are unique to the program 410, so that proofs based on other programs (e.g. modifications of program 410) will not be verified by the verifier 440. In most cases, the trusted third party will destroy the key 415 immediately after the execution of the generator 420.

Any of a number of commercially available zk-SNARK generating systems (compilers) may be used as the generator 420, such as Groth16, Circom, VOProof, Fractal, Halo, SuperSonic, Marlin, ZoKrates, and so on.

As illustrated in FIG. 4B, when subsequently presented with a request for proof, the prover 430 receives as input: the prover key 435, some private data 450, and some public data 460. The private information is commonly referred to as information from a "witness". The private 450 and public 460 data is the information required by the encoding of the statement proving program 410 to prove or disprove the validity of the subject statement.

The prover 430 executes the process corresponding to the encoded program 410, using the prover key 435 and the specific parameters provided in the private 450 and public 460 data, and produces a proof 470. As noted above, the proof 470 is created such that the verifier 440 can ascertain that the prover 430 properly executed the encoded version of the statement proving program 410 and returned a "TRUE" result when the statement was proven to be true, and a "FALSE" result when the statement was not proven to be true. The proof 470, however, does not reveal any of the private data 450 that was used in this TRUE/FALSE determination.

The verifier 440 executes its process using the verifier key 445, the proof 470, and the public data 460. Although the proof 470 does not reveal any information regarding the private data 450, the proof 470 proves to the verifier 440 that the prover 430 had possession of specific private data 450 that, when executed by the encoding of the statement proving program 410, resulted in the returned TRUE/FALSE determination.

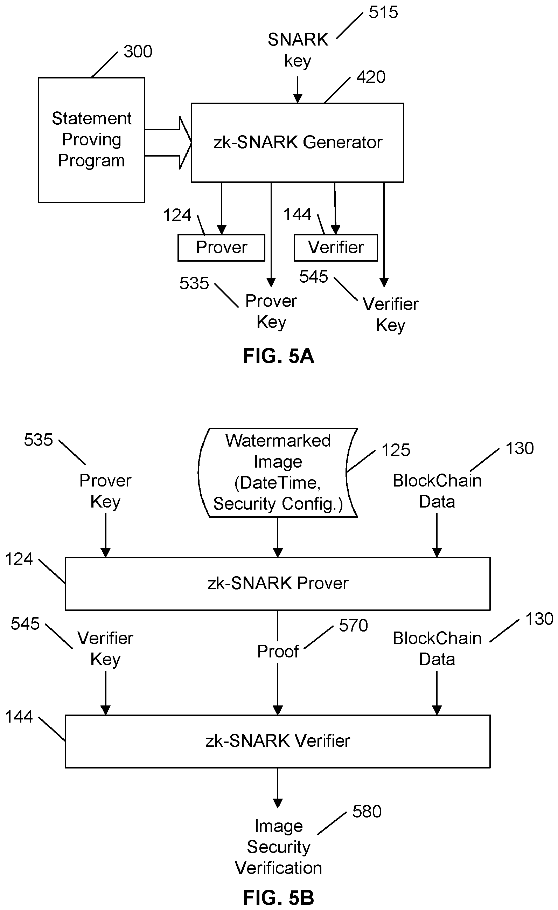

FIGs. 5A-5B illustrate an example zk-SNARK embodiment of an image security configuration verification system in accordance with aspects of this invention using the example elements of FIG. 1, and the example statement proving program 300 of FIG. 3.

In FIG. 5A, the zk-SNARK generator 420, detailed above, receives as input, the statement proving program 300, and a SNARK key 515, and generates the prover 124 and verifier 144, as well as a prover key 535 and a verifier key 545. Operation of the generator 420 using the SNARK key 515 is typically performed by the imaging system provider 110.

As detailed above, the SNARK key 515 assures that the prover 124, verifier 144, prover key 535, and verifier key 545 are unique to the specific encoding of the statement proving program 300 in the prover 124 and corresponding verifier 144. This key 515 is preferably destroyed after the generator 420 is run.

The provider 110 provides the prover 124 and prover key 535 to each imaging facility 120, and provides the verifier 144 and verifier key 545 to the verification system 140.

As noted above with regard to FIGs. 1-2, a user (e.g. patient or agent) 150 submits a request for image security configuration verification to the verification system 140; this request identifies the subject image at a particular imaging facility 120. The verification system 140 submits this request to the imaging facility 120.

The imaging facility 120 retrieves the subject watermarked image 125 from its image database, and submits it to the prover 124.

As illustrated in FIG. 5B, the prover 124 receives the watermarked image 125 (private data), the prover key 535, and accesses the blockchain data 130 (public data) to acquire the records of releases of security configurations from the imaging system provider 110. The prover 124 executes its process using this received data and provides a proof 570 that the security configuration of the imaging system at the date-time of the image (obtained from the watermark of the image) corresponds (or not) to a valid (i.e. up-to-date) security configuration released by the imaging system provider 110, as recorded in the blockchain 130.

The verifier 144 receives this proof 570, its verifier key 545, and accesses the blockchain (public data) 130 to acquire the records of releases of security configurations from the imaging system provider 110. As noted above, the generator 420 creates the prover 124, the verifier 144, the prover key 535, and the verifier key 545 in such a manner that it is virtually impossible for an imaging facility 120 to produce an affirmative proof 570 unless, in fact, the security configuration of the imaging system at the time of acquiring the image, as recorded in the watermarked image, corresponded to a valid security configuration at that time, as recorded by the imaging system provider 110 in the blockchain 130.

In a preferred embodiment, the verification system 140 may be embodied as a "smart contract". As defined by IBM:
"Smart contracts are simply programs stored on a blockchain that run when predetermined conditions are met. They typically are used to automate the execution of an agreement so that all participants can be immediately certain of the outcome, without any intermediary's involvement or time loss. They can also automate a workflow, triggering the next action when conditions are met."³

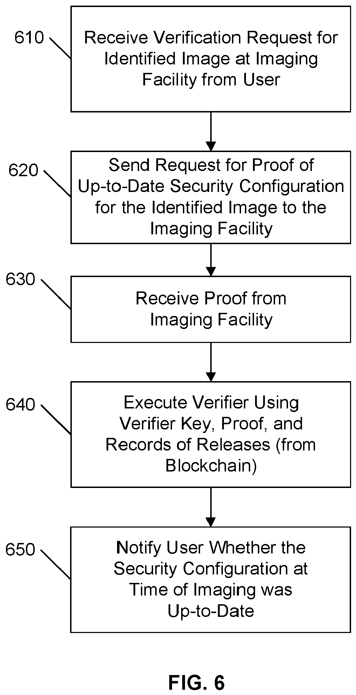

In the context of this invention, with reference to FIGs. 1, 5B, and 6, the smart contract will be structured to receive 610 the user's identification of a watermarked image 125 at an imaging facility 120, and to automatically generate 620 an image security configuration validation request to the imaging facility 120 with the identification of the watermarked image 125. In response, the imaging facility 120 will execute the prover 124, using its prover key 535, the watermarked image 125, and the records of released security configurations on the blockchain 130, and generate the proof 570. The smart contract will receive 630 the proof 570 and execute 640 the verifier 144, using its verifier key 545, the proof 570, and the records of released security configurations on the blockchain 130. The smart contract will return 650 the resultant image security verification, such as, "The identified image was acquired using an 'up-to-date' (or 'out-of-date') security configuration of the imaging system" to the user.

Of particular note, the use of a zk-SNARK embodiment coupled with a smart contract eliminates most, if not all, human interaction (and associated costs) with the operation of the image security configuration verification process of this invention.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### Reference documents:

**¹*CT-GAN: Malicious Tampering of 3D Medical Imagery using Deep Learning,* Mirsky et al., 28^{th} USENIX Security Symposium, 2019.**
**²** The Potential Dangers of Artificial Intelligence for Radiology and Radiologists, Chu et al., Journal of the American College of Radiology: JACR vol. 17,10 (2020): 1309-1311**.**
**³ *What are smart contracts on blockchain*, www.ibm.com/topics/smart-contracts.**

## Claims

1. A verification system comprising:
a user interface that is configured to receive a verification request from a user,
wherein the verification request comprises an identification of an image and an imaging facility,
wherein the verification request comprises a user request to verify that the imaging facility had used an up-to-date security configuration of imaging software when the image was produced,
wherein a provider of the imaging software provides a record of timestamps of releases of each of a plurality of security configurations of the imaging software in a blockchain,
a verification processor that is configured to:
request a proof from the imaging facility that the security configuration of the imaging software was up-to-date when the image was produced,
verify, based on the proof, whether or not the security configuration of the imaging software at the imaging facility was up-to-date when the image was produced,
notify the user of the verification.

2. The verification system of claim 1,
wherein the verification processor comprises a zk-SNARK verifier and an associated verifier key provided by the providing system,
wherein the proof from the imaging facility is a zk-SNARK proof.

3. The verification system of claim 1, wherein the identification of the image comprises a hash of the image.

4. The verification system of claim 1, wherein the verification processor executes a smart contract to request and verify the proof.

5. A system comprising:
an imaging system at an imaging facility,
wherein the imaging facility receives a plurality of security configurations of imaging software for use in the imaging system from a provider system,
wherein each security configuration has a timestamp associated with release of the security configuration from the provider system,
wherein the provider system communicates the timestamp of each security configuration to a storage system,
wherein the storage system is configured to store the timestamp of each security configuration in a blockchain;
wherein, when the imaging system acquires an image of a patient, the imaging system adds a watermark to the image to produce a watermarked image,
wherein the watermark includes an image timestamp and the security configuration of the imaging software used to acquire the image
a processing system at the imaging facility,
wherein the processing system is configured to receive a proof-request from a verification system,
wherein the proof-request includes an identification of a particular image,
wherein the proof-request is a request for a proof from the imaging facility that an up-to-date security configuration of the imaging software was used when the particular image was produced,
wherein the processing system:
decodes the watermark to determine the image timestamp and security configuration used to produce the particular image,
compares the image timestamp and security configuration to the plurality of security configuration timestamps in the blockchain to determine whether the security configuration of the imaging software was up-to-date when the particular image was produced, and
provides proof to the verification system that the security configuration of the imaging software was up-to-date when the particular image was produced.

6. The system of claim 5, wherein the processing system uses a zk-SNARK prover and an associated prover key provided by the providing system to provide the proof to the verification system.

7. The system of claim 5, wherein the identification of the particular image comprises a hash of the particular image.

8. A system comprising:
a compiling system that produces a plurality of security configurations of imaging software over time,
a distribution system that provides each security configuration of the imaging software to at least one imaging facility, and
a storage system that stores a security configuration timestamp corresponding to a release of each security configuration of the imaging software to the imaging facility in a blockchain.

9. The system of claim 8, comprising:
a zk-SNARK setup system that processes a security configuration verification algorithm and generates a zk-SNARK prover, a zk-SNARK prover key, a zk-SNARK verifier, and a zk-SNARK verifier key,
wherein the zk-SNARK prover and zk-SNARK prover key is provided to the imaging facility to provide a proof that the security configuration of the imaging software was up-to-date when a particular image was produced, and
wherein the zk-SNARK verifier and zk-SNARK verifier key is provided to a verification system that verifies the proof provided by the imaging facility.

10. The system of claim 9, wherein the system provides a smart contract to the verification system to facilitate verification that the security configuration of imaging software at the imaging facility was up-to-date when the particular image was produced.
